# EUROPÄISCHE PATENTANMELDUNG

(11) **EP 4 269 345 A1**
(43) Veröffentlichungstag der Anmeldung: **01.11.2023**
(21) Anmeldenummer: 22020194.1
(22) Anmeldetag: 29.04.2022
(51) Int. Cl.: C01C 1/04, B01J 8/02, C07C 29/152

(54) **VERFAHREN UND REAKTOR ZUR KATALYTISCHEN UMSETZUNG EINES EINSATZSTROMS**

(71) Anmelder: Linde GmbH, 82049 Pullach (DE)
(72) Erfinder: Mihailowitsch, Dieter, 82049 Pullach (DE); Zander, Hans-Jörg, 82049 Pullach (DE); Stefanescu, Adriana, 82049 Pullach (DE); Herzog, Robert, 82049 Pullach (DE)
(74) Vertreter: Fischer, Werner

(57) **Zusammenfassung**

Die vorliegende Erfindung betrifft ein Verfahren zur katalytischen Umsetzung eines Einsatzstroms (1) unter Erhalt zumindest eines Produktstroms (3), wobei die katalytische Umsetzung in einem Katalysatorbett (140) durchgeführt wird und der Einsatzstrom (1) zur Kühlung des Katalysatorbetts (140) verwendet wird, dadurch gekennzeichnet, dass der Einsatzstrom (1) während seiner Verwendung zur Kühlung des Katalysatorbetts (140) abwechselnd in einer ersten (11) und einer zweiten (12) Richtung an dem Katalysatorbett (140) vorbeigeleitet wird, dass der Einsatzstrom (1) während seiner katalytischen Umsetzung zu dem Produktstrom (3) in einer dritten Richtung (13) durch das Katalysatorbett (140) geleitet wird, dass die zweite Richtung (12) der ersten Richtung (11) entgegengesetzt verläuft und die dritte Richtung (13) weder der ersten (11) noch der zweiten (12) Richtung entspricht, und dass der Einsatzstrom (1) während des Wechsels von der ersten (11) in die zweite (12) Richtung und von der zweiten (12) in die erste (11) Richtung zumindest einmal in der dritten Richtung (13) und zumindest einmal entgegen der dritten Richtung (13) versetzt wird. Ferner wird ein entsprechender Reaktor vorgeschlagen.

## Beschreibung

Die Erfindung betrifft ein Verfahren sowie einen Reaktor zur katalytischen Umsetzung eines Einsatzstroms, insbesondere zur Methanol- und/oder Ammoniaksynthese.

In Anlagen und Verfahren zur katalytischen Umsetzung eines Einsatzstroms kann der jeweilige Einsatzstrom im Gegenstrom gegen einen den Katalysator verlassenden Produktstrom erwärmt werden, um den Einsatzstrom auf eine Temperatur zu bringen, bei der der jeweilige Katalysator effizient arbeiten kann.

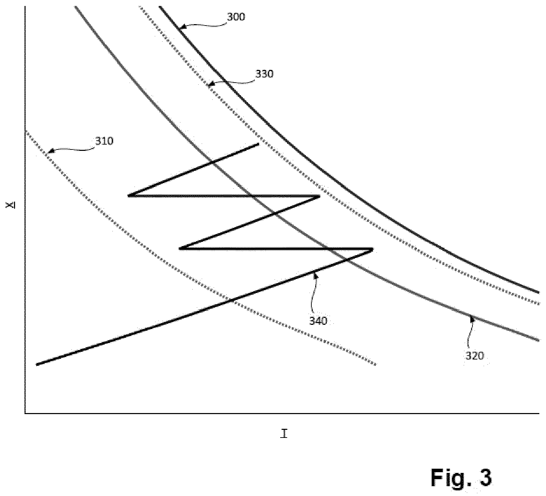

Dabei können auch Zwischenkühlungen vorgesehen sein, so dass beispielsweise das jeweilige Katalysatorbett auf drei Einzelreaktoren aufgeteilt ist und jeweils zwischen den Reaktoren ein Wärmetausch zwischen dem jeweiligen (Zwischen-)Produkt und dem Einsatzstrom vorgesehen werden kann. Dadurch wird eine gleichmäßigere Wärmetönung angestrebt und es werden Gleichgewichtslimitierungen vermieden. Ein mittels derartiger Zwischenkühlungen erreichbares Temperaturverhalten ist in Fig. 3 vereinfacht in Form eines Diagramms des Reaktionsfortschritts X über der Temperatur T dargestellt, die hier zu Erläuterungszwecken vorab beschrieben werden soll. In dem X/T Diagramm ist der Verlauf des thermodynamischen Gleichgewichts einer beispielhaften Reaktion einer Ammoniaksynthese gezeigt (Kurve 300). Die optimale Reaktionsführung in Bezug auf die Reaktionsgeschwindigkeit ist mit der Kurve 320 gezeigt. 310 und 330 bezeichnen jeweils Verläufe mit der halben Reaktionsgeschwindigkeit im Vergleich zu dem optimalen Verlauf 320. Die Kurve 340 illustriert demgegenüber einen typischen Reaktionsverlauf, wie er im Rahmen üblicher Verfahren mit Zwischenkühlungen (waagerechte Teilbereiche der Kurve 340) beobachtet wird. Dabei ist erkennbar, dass sich die Kurve 340 zumindest bei einer kleinen Stufenzahl von der optimalen Kurve 320 entfernt.

Dementsprechend besteht bezüglich der Temperaturverteilung innerhalb des gesamten Katalysatorbetts noch erheblicher Verbesserungsbedarf, so dass Verfahrensweisen und Anlagen gesucht werden, bei denen eine möglichst günstige Temperaturverteilung erreicht wird.

Diese Aufgabe wird durch Verfahren und Reaktoren gemäß den jeweiligen unabhängigen Patentansprüchen gelöst. Vorteilhafte Ausgestaltungen und Weiterbildungen sind jeweils Gegenstand der abhängigen Patentansprüche sowie der vorliegenden Beschreibung.

Bei einem erfindungsgemäßen Verfahren zur katalytischen Umsetzung eines Einsatzstroms unter Erhalt zumindest eines Produktstroms, werden die katalytische Umsetzung in einem Katalysatorbett durchgeführt und der Einsatzstrom zur Kühlung des Katalysatorbetts verwendet, wobei der Einsatzstrom während seiner Verwendung zur Kühlung des Katalysatorbetts abwechselnd in einer ersten und einer zweiten Richtung an dem Katalysatorbett vorbeigeleitet wird, wobei der Einsatzstrom während seiner katalytischen Umsetzung zu dem Produktstrom in einer dritten Richtung durch das Katalysatorbett geleitet wird, wobei die zweite Richtung der ersten Richtung entgegengesetzt verläuft und die dritte Richtung weder der ersten noch der zweiten Richtung entspricht, und wobei der Einsatzstrom während des Wechsels von der ersten in die zweite Richtung und während des Wechsels von der zweiten in die erste Richtung auf seinem Weg vorbei am Katalysatorbett zumindest einmal in der dritten Richtung und zumindest einmal entgegen der dritten Richtung versetzt wird. Mit anderen Worten wird der Einsatzstrom nach Durchlaufen der ersten (oder zweiten) Richtung und vor Durchlaufen der zweiten (oder ersten) Richtung einmal in der dritten Richtung versetzt und nach Durchlaufen der ersten (oder zweiten) Richtung und vor Durchlaufen der zweiten (oder ersten) Richtung einmal entgegen der dritten Richtung versetzt.

Es versteht sich, dass die erste bzw. die zweite Richtung auch außerhalb des Katalysatorbettes durchlaufen werden kann, also der Einsatzstrom beispielsweise nur in der ersten Richtung an dem Katalysatorbett vorbeigeführt wird, um dieses zu kühlen, und außerhalb des Katalysatorbettes (also ohne Kühlwirkung auf dieses) wieder zu der Eingangsseite zurückgeführt wird, bevor er erneut in der ersten Richtung an dem Katalysatorbett kühlend vorbeiströmt. Eine solche Konfiguration kann selbstverständlich auch nur für einen Teil der Durchläufe verwendet werden.

Viele chemische Reaktionen verlaufen umso schneller, je höher die Temperatur am Reaktionsort ist. Gleichzeitig wird durch eine Temperaturerhöhung das Reaktionsgleichgewicht einer exothermen Reaktion auf die Eduktseite verschoben, so dass bei einer zu hohen Temperatur eine Gleichgewichtslimitierung des Umsatzes eintritt. Es ergibt sich folglich ein Optimierungsproblem, bei dem das Katalysatorbett möglichst präzise in einem schmalen Temperaturkorridor gehalten werden sollte, um maximale Prozessausbeute zu ermöglichen. Dabei ist ferner zu beachten, dass es innerhalb des Katalysatorbetts nicht eine konstante Optimaltemperatur gibt, sondern sich dieses Temperaturoptimum aufgrund der sich verändernden Konzentrationsverhältnisse, die sich über die Reaktionsstrecke durch die fortschreitende Reaktion zwangsläufig ergeben, örtlich verändert, wie auch aus der eingangs erläuterten Figur 3 ersichtlich.

Es versteht sich, dass der Einsatzstrom, bevor er zur Kühlung des Katalysatorbetts verwendet wird, eine im Vergleich zu dem Katalysatorbett niedrige Temperatur aufweist. Daraus ergibt sich, dass an einer Eintrittsstelle des Einsatzstroms in das Katalysatorbett eine hohe Temperaturdifferenz und damit eine starke Kühlwirkung auftritt, da der Einsatzstrom an dieser Stelle noch nicht erwärmt wurde. Herkömmlicherweise wird bei einer Gegenstromkühlung der Einsatzstrom entgegengesetzt zu dem zu kühlenden Produktstrom geführt, um eine möglichst hohe Kühlwirkung zu erzielen. Im Gegensatz dazu sieht die Erfindung vor, dieses Gegenstromprinzip zu durchbrechen, um eine möglichst nahe am reaktionskinetischen Optimum liegende Temperaturverteilung in dem Katalysatorbett zu erreichen. Dazu wird die Kühlung einerseits direkt in dem Katalysatorbett realisiert, und nicht der Produktstrom erst nach Verlassen des Katalysatorbetts gekühlt. Andererseits wird der kühlende Einsatzstrom nicht im Gegenstrom durch das Katalysatorbett geführt, sondern, wie bereits erwähnt, entlang der ersten und zweiten Richtung hin und her, während er bei einem Wechsel von der ersten in die zweite Richtung und umgekehrt entlang bzw. entgegengesetzt der dritten Richtung, die der Strömungsrichtung in dem Katalysatorbett entspricht, versetzt wird.

In einem Beispielsfall, bei dem die erste und die zweite Richtung im Wesentlichen horizontal und die dritte Richtung im Wesentlichen vertikal gerichtet sind, kann der Einsatzstrom von links kommend in einer ersten Höhe zur Kühlung an dem Katalysatorbett vorbeigeführt werden, dann in einer zweiten Höhe, z.B. oberhalb der ersten Höhe, wieder nach links zurückgeführt werden und anschließend in einer dritten Höhe, die in diesem Beispiel entweder zwischen der ersten und der zweiten Höhe oder unterhalb der ersten Höhe liegt, wieder von links nach rechts an dem Katalysatorbett vorbei geführt werden. Stromab dieser dritten Vorbeiführung wird der nun erwärmte Einsatzstrom in der dritten Richtung, zum Beispiel von oben, in das Katalysatorbett geleitet. Dadurch ergibt sich eine zwar unregelmäßig erscheinende Kühlung, die jedoch genau so gestaltet werden kann, dass die Temperatur des Katalysatorbetts über die gesamte Reaktionsstrecke möglichst nahe an der jeweils örtlichen Optimaltemperatur liegt.

Die dritte Richtung verläuft bevorzugt senkrecht zu der ersten und zweiten Richtung. Dies ist einerseits konstruktionstechnisch günstig, andererseits erleichtert es die Ermittlung der thermisch sinnvollsten Reihenfolge an Richtungswechseln des zur Kühlung verwendeten Einsatzstroms.

Insbesondere verläuft die dritte Richtung geodätisch senkrecht, insbesondere nach geodätisch unten. Dadurch wird einem durch Konvektion verursachten Temperaturgradienten vorgebeugt.

Die katalytische Umsetzung umfasst vorteilhafterweise eine oder mehrere chemische Reaktionen zur Synthese von Ammoniak und/oder Methanol. Dies sind besonders relevante Anwendungsfälle, in denen dieses vom Gegenstromprinzip abweichende Kühlregime vorteilhaft eingesetzt werden kann.

Ein erfindungsgemäßer Reaktor zur katalytischen Umsetzung eines Einsatzstroms zu einem Produktstrom umfasst ein Katalysatorbett und Wärmetauscherrohre bzw. andere Wärmetauschelemente wie z.B. Plattenpaare (im Folgenden der besseren Lesbarkeit halber, jedoch ausdrücklich ohne Beschränkung der Allgemeinheit dieses Begriffs, nur als Wärmetauscherrohre bezeichnet), die durch das Katalysatorbett verlaufen, wobei die Wärmetauscherrohre in zumindest erste, zweite und dritte Rohrsätze (bzw. Wärmetauschelementsätze, im Folgenden kurz: Rohrsätze) unterteilt sind, die jeweils parallel zueinander angeordnet sind, wobei jeder der ersten, zweiten und dritten Rohrsätze jeweils ein Eingangs- und ein Ausgangsende aufweist, und wobei das Ausgangsende des ersten Rohrsatzes mit dem Eingangsende des zweiten Rohrsatzes verbunden ist und das Ausgangsende des zweiten Rohrsatzes mit dem Eingangsende des dritten Rohrsatzes verbunden ist. Ein erfindungsgemäßer Reaktor ist dadurch gekennzeichnet, dass der zweite Rohrsatz in dem Katalysatorbett nicht zwischen dem ersten und dem dritten Rohrsatz angeordnet ist und dass stromab des dritten Rohrsatzes die Wärmetauscherrohre einen Ausgang in das Katalysatorbett aufweisen. Dadurch wird ein vom Gegenstromprinzip abweichendes Kühlregime mit den bereits erläuterten Vorteilen ermöglicht.

Insbesondere ist der Reaktor zur Durchführung eines Verfahrens wie vorstehend beschrieben eingerichtet und profitiert daher von dessen Vorteilen in analoger Weise.

Vorteilhafterweise sind in dem Katalysatorbett stromab des dritten Rohrsatzes und stromauf des Ausgangs in das Katalysatorbett zumindest ein weiterer Rohrsatz, beispielsweise ein vierter, fünfter etc. Rohrsatz, parallel zu dem ersten, zweiten und dritten Rohrsatz angeordnet. Dies erweitert die Möglichkeiten der präzisen Temperatureinstellung sowie insgesamt die Wärmetauschleistung. Hierbei kann der vierte Rohrsatz beispielsweise direkt benachbart zum dritten Rohrsatz angeordnet sein. Ferner kann ein weiterer (fünfter) Rohrsatz direkt benachbart zum vierten Rohrsatz angeordnet sein. Solche Anordnungen werden in den Ausführungsbeispielen näher erläutert.

Bevorzugt umfasst der Reaktor eine Reaktorhülle und eine aus der Reaktorhülle entnehmbare Kartusche, wobei die Kartusche zumindest das Katalysatorbett und die Wärmetauscherrohre umfasst. Dadurch kann das Katalysatorbett einfach und zeitsparend ausgetauscht werden, so dass sich für diese regelmäßig erforderliche Maßnahme nur eine geringe Anlagenausfallzeit ergibt und damit insgesamt eine bessere Anlagenauslastung erzielt werden kann. Der (insbesondere zylindrische) Mantel nimmt die Druckdifferenz zwischen Verfahrensdruck und Umgebung auf, während die internen Strukturen des Reaktors nicht durch den Verfahrensdruck belastet werden.

Insbesondere ist dabei in der Reaktorhülle zumindest eine Schiene vorgesehen, entlang derer die Kartusche in die Reaktorhülle eingeführt werden kann. Dies erleichtert die Montage und den Austausch der Kartusche und verringert damit die dafür benötigte Zeit noch weiter.

Vorteilhafterweise umfasst dabei die Kartusche zumindest ein Einführelement, wobei das Einführelement zum Zusammenwirken mit der zumindest einen Schiene eingerichtet ist. Damit kann die Kartusche sicher entlang der Schiene geführt werden. Das Katalysatorbett umfasst bevorzugt zumindest einen Katalysator, der zur Katalyse zumindest einer chemischen Reaktion zur Synthese von Ammoniak und/oder Methanol in der Lage ist. Diese Synthesen sind für die beschriebene Kühlung, die vom bekannten Gegenstromprinzip abweicht, besonders relevant und geeignet.

### Kurze Beschreibung der Figuren

Figur 1 zeigt schematisch eine vorteilhafte Ausgestaltung der Erfindung.
Figur 2 zeigt eine detailliertere schematische Darstellung eines Teils der vorteilhaften Ausgestaltung der Erfindung gemäß Figur 1.
Figur 3 zeigt einen typischen Temperaturverlauf in einem Reaktor herkömmlicher Art.
Figur 4 zeigt einen typischen Temperaturverlauf, wie er im Rahmen von vorteilhaften Ausgestaltungen der Erfindung beobachtet werden kann.

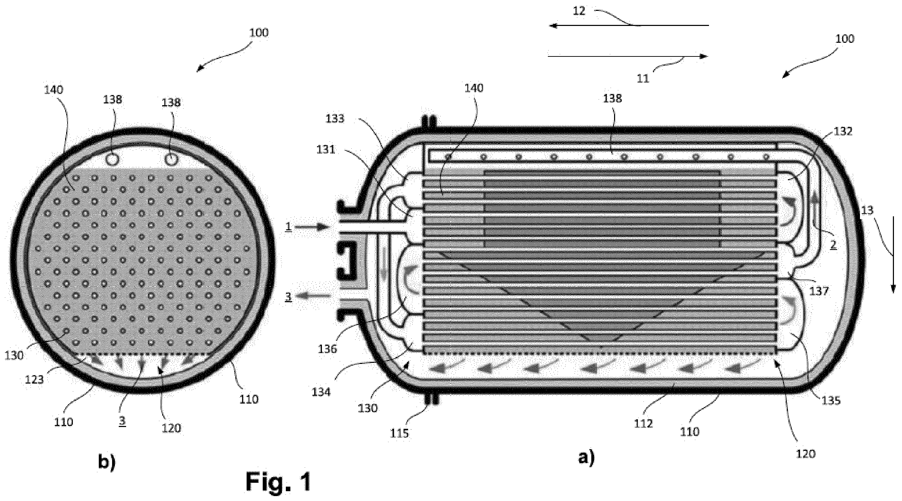

In Figur 1 ist ein Reaktor gemäß einer vorteilhaften Ausgestaltung der Erfindung schematisch dargestellt und insgesamt mit 100 bezeichnet. Dabei ist eine Seitenansicht a) sowie eine Ansicht b) in Richtung einer Achse des Reaktors 100, jeweils im Schnitt, dargestellt.

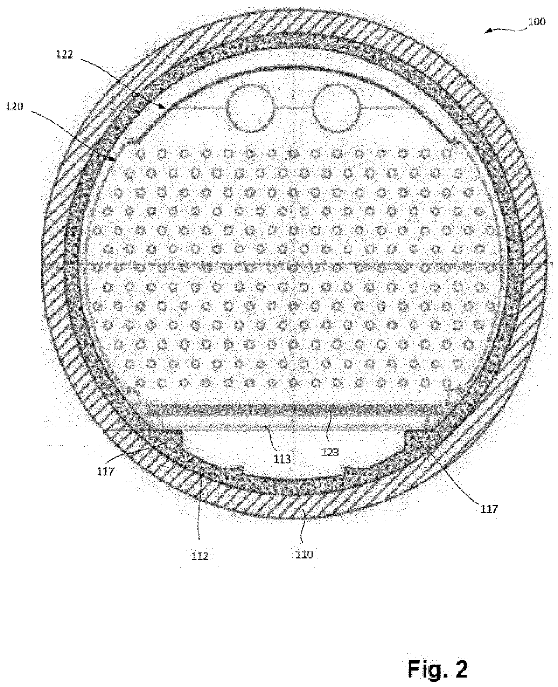

Der Reaktor 100 weist eine im Wesentlichen zylindrische Reaktorhülle 110, einen Rohrwärmetauscher 130 sowie ein Katalysatorbett 140 auf. Wärmetauscher 130 und Katalysatorbett 140 sind in dem gezeigten Beispiel zu einer Kartusche 120 zusammengefasst, die aus der Reaktorhülle 110 entnehmbar ist, wie in Figur 2 gezeigt und unten näher beschrieben ist. Die Ausgestaltung der Figur 2 entspricht dabei im Wesentlichen der aus Figur 1, wobei die Darstellung in Figur 2 detaillierter ist.

Die Reaktorhülle 110 ist mit einer Isolierung 112 ausgekleidet, um thermische Verluste zu minimieren und den drucktragenden Mantel von der Verfahrenstemperatur zu entlasten. Ferner weist die Reaktorhülle 110 einen Flansch 115 auf, mittels dessen sie zusammengehalten wird. Zur Entnahme bzw. zum Einbau der Kartusche 120 kann die Reaktorhülle 110 entlang des Flansches 115 geöffnet werden. Im geodätisch unteren Teil der Reaktorhülle 110 sind Schienen 117 vorgesehen, auf denen die Kartusche 120 ruht und im Falle eines Austauschs gleiten bzw. rollen kann. Dies erleichtert Zusammenbau und Wartung des Reaktors 100 erheblich. Während des Betriebs des Reaktors 100 ist die Kartusche 120 auf den Schienen 117 derart gelagert, dass ihre Position fixiert ist. So kann eine Beschädigung des Reaktors 100 verhindert werden. Zur weiteren Vereinfachung der Wartung können an der Kartusche 120 ein oder mehrere Einführelemente, die zur Zusammenwirkung mit der Schiene 117 eingerichtet sind, vorgesehen sein, beispielsweise in der Form von Rädern oder Kufen. Die Kartusche 120 ist an ihrem unteren Ende durch einen fluiddurchlässigen Boden 123 begrenzt, der den Katalysator 140 zurückhält, Reaktionsprodukte 3 jedoch passieren lässt. Beispielsweise kann der Boden 123 in Form eines Rostes oder in Form von Gazematten bereitgestellt werden, wobei auch andere geeignete Ausgestaltungen verwendet werden können. Der Boden 123 wird von einer Stützstruktur 113 gestützt, beispielsweise in Form eines Tragrahmens, die verhindert, dass der Boden 123 zu stark durchhängt, insbesondere bei hohen Temperaturen. In bevorzugten Ausgestaltungen ist der Boden 123 der Kartusche 120 von unten her demontierbar, um eine einfache Entnahme des Katalysatorbettes 140, beispielsweise zur Erneuerung von verbrauchtem Katalysator, zu ermöglichen. An der oberen Seite ist die Kartusche über einen abnehmbaren Deckel 122 abgedichtet, um das Füllen bzw. Entleeren der Kartusche 120 mit Katalysator 140 zu ermöglichen.

Der Rohrwärmetauscher 130 ist so angeordnet, dass die Wärmetauscherrohre, die den Wärmetauscher 130 bilden, parallel zu der Achse des Reaktors 100 verlaufen. In dem gezeigten Beispiel ist der Wärmetauscher 130 in fünf Rohrsätze unterteilt, die jeweils parallel zueinander übereinander durch das Katalysatorbett 140 verlegt sind.

Der Wärmetauscher 130 wird beim Betrieb des Reaktors 100 von einem Einsatzstrom 1 durchströmt, der dabei erwärmt wird, während das Katalysatorbett 140 gekühlt wird. Der erwärmte Einsatzstrom 2 tritt durch einen Ausgang 138 des Wärmetauschers 130 in das Katalysatorbett aus und wird beim Durchströmen des Katalysatorbetts 140 in einer Richtung (in Figur 1 durch einen breiten Pfeil angedeutet), die im Wesentlichen senkrecht zu der Achse des Reaktors 100 verläuft, zu einem Produktstrom 3 umgewandelt.

Das Besondere des Reaktors 100 ist hierbei die Reihenfolge bzw. Schichtung der fünf Rohrsätze des Wärmetauschers 130. Diese weicht deutlich von einem klassischen Gegenstromprinzip ab, da der kalte Einsatzstrom nicht an einem Ausgang des Katalysatorbetts in den Wärmetauscher 130 einströmt, sondern - in dem gezeigten Beispiel - von der Mitte des Katalysatorbetts 140 etwas in Richtung des Eingangs des Katalysatorbetts 140 (bzw. in Richtung des Ausgangs 138 des Wärmetauschers 130) versetzt. Dazu wird der Einsatzstrom über einen Verteilereingang 131 des ersten Rohrsatzes auf die einzelnen Rohre des ersten Rohrsatzes aufgeteilt. Nach Durchlaufen des ersten Rohrsatzes in einer ersten Richtung 11 wird der Einsatzstrom 1 in einem Sammelverteiler 132, der zugleich Ausgang des ersten Rohrsatzes und Eingang des zweiten Rohrsatzes ist, in eine zweite Richtung 12, die der ersten Richtung 11 im Wesentlichen entgegengesetzt ist, umgelenkt und in den zweiten Rohrsatz, der in direkter Nachbarschaft zum Eingang des Katalysatorbetts angeordnet ist, geleitet.

Am Ausgang des zweiten Rohrsatzes wird des Einsatzstrom 1 wiederum in einem Sammler 133 gesammelt und zu einem Verteiler 134, der den Eingang des dritten Rohrsatzes darstellt, in eine dritte Richtung 13 geleitet. Der dritte Rohrsatz ist in dem hier gezeigten Beispiel am Ausgang des Katalysatorbetts 140 angeordnet, wird wiederum in der ersten Richtung 11 durchströmt und mündet in einen weiteren Sammelverteiler 135, von dem aus der Einsatzstrom in den vierten Rohrsatz, der direkt benachbart zu dem dritten Rohrsatz angeordnet ist und wiederum in der zweiten Richtung 12 durchströmt wird, umgeleitet wird. Der vierte Rohrsatz mündet wiederum in einem Sammelverteiler 136, der den Einsatzstrom in den fünften Rohrsatz, der hier abermals in der ersten Richtung 11 zwischen dem ersten und dem vierten Rohrsatz angeordnet ist, umleitet, an dessen Ausgang der nun erwärmte Einsatzstrom 2 abermals in einem Sammler zusammengefasst und zu dem Ausgang 138 des Wärmetauschers 130 geleitet wird.

Das Katalysatorbett wird hingegen in einer dritten Richtung 13, die im Wesentlichen senkrecht zu der ersten 11 und zweiten 12 Richtung steht, durchströmt, wobei allfällige Turbulenzen bei der Bestimmung der Strömungsrichtung außer Acht gelassen werden und lediglich die resultierende Hauptströmungsrichtung betrachtet wird.

Der Produktstrom 3 wird dem Reaktor 100 in dem gezeigten Beispiel axial entnommen, ebenso wie der Einsatzstrom 1 dem Reaktor 100 axial zugeführt wird.

Durch die nicht dem klassischen Gegenstromprinzip entsprechende Medienführung wird eine Temperaturverteilung in dem Katalysatorbett 140 erreicht, die im Vergleich zu der eingangs erläuterten Temperaturverteilung herkömmlicher Systeme mit Zwischenkühlung erheblich näher an der optimalen Temperaturkurve 320 liegt. Dadurch kann eine Verkürzung der Verweilzeit im Reaktor bzw. ein höherer Durchsatz erzielt werden, was die Effizienz der Anlage insgesamt erhöht.

Es versteht sich, dass je nach Anwendung und Dimensionierung des Reaktors 100 auch mehr oder weniger Rohrsätze vorgesehen werden können. Die Abweichung von dem klassischen Gegenstromprinzip und eine Schichtung der einzelnen Rohrbündel zueinander zur Erzielung des jeweils möglichst nahe am reaktionskinetischen Optimum liegenden Temperaturprofils innerhalb des Katalysatorbetts 140 sind dabei das Wesentliche der vorliegenden Erfindung. Vorteilhaft sind solche Reaktoren prinzipiell für alle exothermen Reaktionen, die bei hohen Temperaturen ablaufen und durch das thermische Gleichgewicht limitiert sind. Durch die kontinuierliche Kühlung kann eine höhere durchschnittliche Reaktortemperatur realisiert werden, ohne eine thermische Überlastung des Katalysators befürchten zu müssen.

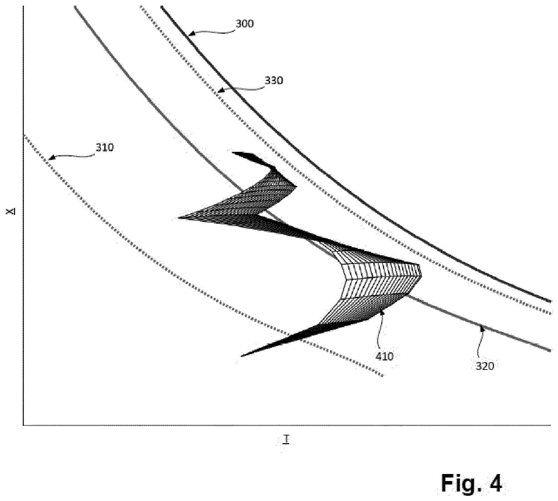

In Figur 4 ist beispielhaft ein Reaktionsverlaufsdiagramm, wie es im Rahmen der Erfindung beobachtet werden kann, dargestellt. Analog zu dem Diagramm aus Figur 3 ist auch hier der Reaktionsfortschritt X über der Temperatur T mit den jeweils identischen Vergleichskurven 300, 310, 320, 330 dargestellt. Dabei ist zu beachten, dass hierbei eine nicht einheitliche Temperaturverteilung über die gesamte Länge des Reaktors 100 beobachtet wird, da sich der Einsatzstrom 1 über die Länge des Reaktors 100 erwärmt. Dennoch ist klar erkennbar, dass sich die Kurvenschar 410, die den Reaktionsverlauf beschreibt, in Figur 4 über weite Bereiche des Reaktionsgeschehens näher an die Optimalkurve 320 anschmiegt, als dies für die Kurve 340 in Figur 3 der Fall ist. Dementsprechend ergibt sich durch die Erfindung eine im Vergleich zum Stand der Technik verbesserte Reaktionsführung mit den entsprechenden Vorteilen in Bezug auf Prozessausbeute bzw. Energie- und Ressourceneffizienz.

Unabhängig von der konkreten Ausgestaltung des Reaktors 100 kann es vorteilhaft sein, zwischen zwei aufeinanderfolgenden Rohrsätzen einen Teilstrom des jeweiligen Einsatzstroms zu entnehmen und/oder einen zusätzlichen Teilstrom zuzuführen. Das kann insbesondere ein temperierter Einsatzstrom sein, mit dessen Eintrittstemperatur das Temperaturprofil des Reaktors eingestellt werden kann, bevorzugt ein vergleichsweise kalter Einsatzstrom, über dessen Einmischung der Reaktor gekühlt wird.

In einigen Ausgestaltungen kann der Reaktor 100 im Teillastbetrieb derart betrieben werden, dass ein Teil des Produktstroms 3 als Recycle in den Reaktor 100 zurückgeführt wird und gleichzeitig dessen Eintrittstemperatur so gesetzt wird, dass in den beiden oberen Rohrsätzen - bzw. allgemeiner formuliert in den Rohrsätzen, die geometrisch zwischen dem rohseitigen Eintritt (Verteiler 131) und dem Festbetteintritt 138 liegen - ein für den gewünschten Umsatz ausreichendes Reaktionsgeschehen stattfindet. Dazu kann eine höhere bzw. niedrigere, auch deutlich höhere bzw. niedrigere, Eintrittstemperatur als am normalen Betriebspunkt eingestellt werden. Beispielsweise kann die Temperatur im Teillastbetrieb mindestens 10 °C, 20 °C oder bis zu 50 °C über oder unter der für diese Reaktionszone als optimal ermittelte Reaktionstemperatur liegen. Eine höhere Konzentration des Produkts, beispielsweise Ammoniak, im Einsatzstrom 1 kann beispielsweise eingestellt werden, indem in einem der Reaktion in dem Reaktor 100 nachgelagerten Prozess zur Aufreinigung des Produktstroms 3 eine höhere Kondensationstemperatur als im Normalbetrieb gewählt wird oder indem ein Teilstrom des Produktstroms unter Umgehung eines dazu verwendeten Kondensators recycelt wird, so dass eine geringere Menge des Zielprodukts kondensiert, bevor man das verbleibende Gas zum Reaktor recycelt. Durch die so erreichte höhere Produktkonzentration im Einsatzstrom 1 wird das Reaktionsgleichgewicht in Richtung der Edukte verlagert, was die Reaktionsgeschwindigkeit insgesamt bremst. Damit kann gezielt die angestrebte Teillast eingestellt werden.

Es sei an dieser Stelle ausdrücklich betont, dass im Rahmen der vorliegenden Erfindung unter dem Begriff "erster Rohrsatz" nicht zwingend der tatsächlich als eingangsseitig zuerst durchlaufene Rohrsatz zu verstehen ist. Beispielsweise kann der Einsatzstrom 1, bevor er den "ersten Rohrsatz" durchströmt, bereits einen oder mehrere weitere Rohrsätze durchströmt haben. Analoges gilt selbstverständlich für die Begriffe "zweiter Rohrsatz" und "dritter Rohrsatz". Die Bezeichnung ist vielmehr als Unterscheidungsmerkmal zu verstehen, um die erfindungsrelevante Reihenfolge der Durchströmung der ersten, zweiten und dritten Rohrsätze zu bezeichnen.

Ferner sei hier nochmals darauf hingewiesen, dass anstatt von Wärmetauscherrohren 130 auch andere Wärmetauscherelemente, wie beispielsweise Plattenpaare eines Plattenwärmetauschers, verwendet werden können. Die vorstehende Beschreibung mit Bezug auf Rohrwärmetauscher ist demgemäß lediglich als beispielhafte Ausführungsform zu verstehen.

## Patentansprüche

1. Verfahren zur katalytischen Umsetzung eines Einsatzstroms (1) unter Erhalt zumindest eines Produktstroms (3),
wobei die katalytische Umsetzung in einem Katalysatorbett (140) durchgeführt wird und der Einsatzstrom (1) zur Kühlung des Katalysatorbetts (140) verwendet wird, **dadurch gekennzeichnet,**
**dass** der Einsatzstrom (1) während seiner Verwendung zur Kühlung des Katalysatorbetts (140) abwechselnd in einer ersten (11) und einer zweiten (12) Richtung an dem Katalysatorbett (140) vorbeigeleitet wird,
**dass** der Einsatzstrom (1) während seiner katalytischen Umsetzung zu dem Produktstrom (3) in einer dritten Richtung (13) durch das Katalysatorbett (140) geleitet wird,
**dass** die zweite Richtung (12) der ersten Richtung (11) entgegengesetzt verläuft und die dritte Richtung (13) weder der ersten (11) noch der zweiten (12) Richtung entspricht, und
**dass** der Einsatzstrom (1) während des Wechsels von der ersten (11) in die zweite (12) Richtung und von der zweiten (12) in die erste (11) Richtung zumindest einmal in der dritten Richtung (13) und zumindest einmal entgegen der dritten Richtung (13) versetzt wird.

2. Verfahren nach Anspruch 1, wobei die dritte Richtung (13) senkrecht zu der ersten (11) und zweiten (12) Richtung verläuft.

3. Verfahren nach Anspruch 1 oder 2, wobei die dritte Richtung (13) geodätisch senkrecht, insbesondere nach geodätisch unten, verläuft.

4. Verfahren nach einem der vorstehenden Ansprüche, wobei die katalytische Umsetzung eine oder mehrere chemische Reaktionen zur Synthese von Ammoniak und/oder Methanol umfasst.

5. Verfahren nach einem der vorstehenden Ansprüche, wobei ein Teil des Produktstroms (3) in einem Teillastbetrieb als Recycle in den Einsatzstrom (1) zurückgeführt wird und/oder die Eintrittstemperatur des Einsatzstroms (1) in Abhängigkeit von einer gewünschten Reaktionsgeschwindigkeit eingestellt wird.

6. Reaktor (100) zur katalytischen Umsetzung eines Einsatzstroms (1) zu einem Produktstrom (3), umfassend ein Katalysatorbett (140) und Wärmetauschelemente (130), die durch das Katalysatorbett (140) verlaufen,
wobei die Wärmetauschelemente (130) in zumindest erste, zweite und dritte Wärmetauschelementsätze unterteilt sind, die jeweils parallel zueinander angeordnet sind,
wobei jeder der ersten, zweiten und dritten Wärmetauschelementsätze jeweils ein Eingangs- (131, 132, 134, 135, 136) und ein Ausgangsende (132, 133, 135, 136, 137) aufweist, und
wobei das Ausgangsende des ersten Wärmetauschelementsatzes (132) mit dem Eingangsende des zweiten Wärmetauschelementsatzes (132) verbunden ist und das Ausgangsende des zweiten Wärmetauschelementsatzes (133) mit dem Eingangsende des dritten Wärmetauschelementsatzes (134) verbunden ist,
**dadurch gekennzeichnet,**
**dass** der zweite Wärmetauschelementsatz in dem Katalysatorbett (140) nicht zwischen dem ersten und dem dritten Wärmetauschelementsatz angeordnet ist und
**dass** stromab des dritten Wärmetauschelementsatzes die Wärmetauschelemente (130) einen Ausgang (138) in das Katalysatorbett (140) aufweisen.

7. Reaktor (100) nach Anspruch 6, wobei der Reaktor (100) zur Durchführung eines Verfahrens nach einem der Ansprüche 1 bis 5 eingerichtet ist.

8. Reaktor (100) nach Anspruch 6 oder 7, wobei in dem Katalysatorbett (140) stromab des dritten Wärmetauschelementsatzes und stromauf des Ausgangs (138) in das Katalysatorbett (140) zumindest ein weiterer Wärmetauschelementsatz parallel zu dem ersten, zweiten und dritten Wärmetauschelementsatz angeordnet ist.

9. Reaktor (100) nach einem der Ansprüche 6 bis 8 mit einem oder mehreren Fluidanschlüssen, der bzw. die dazu eingerichtet sind, einen oder mehrere Teilströme zwischen zwei aufeinander folgenden Wärmetauschelementsätzen zu entnehmen und/oder zuzuführen.

10. Reaktor (100) nach einem der Ansprüche 6 bis 9, umfassend eine Reaktorhülle (110) und eine aus der Reaktorhülle (110) entnehmbare Kartusche (120), wobei die Kartusche (120) zumindest das Katalysatorbett (140) und die Wärmetauschelemente (130) umfasst.

11. Reaktor (100) nach Anspruch 10, wobei in der Reaktorhülle (110) zumindest eine Schiene (117) vorgesehen ist, entlang derer die Kartusche (120) in die Reaktorhülle (110) eingeführt werden kann.

12. Reaktor (100) nach Anspruch 11, wobei die Kartusche (120) zumindest ein Einführelement umfasst, wobei das zumindest eine Einführelement zum Zusammenwirken mit der zumindest einen Schiene (117) eingerichtet ist.

13. Reaktor (100) nach einem der Ansprüche 10 bis 12, wobei die Kartusche (120) oben mit einem abnehmbaren Deckel (122) abgedichtet ist.

14. Reaktor (100) nach einem der Ansprüche 10 bis 13, wobei die Kartusche (120) unten durch einen, insbesondere von unten demontierbaren, fluiddurchlässigen Boden (123) begrenzt ist.

15. Reaktor (100) nach einem der Ansprüche 6 bis 14, wobei das Katalysatorbett (140) zumindest einen Katalysator umfasst, der zur Katalyse zumindest einer chemischen Reaktion zur Synthese von Ammoniak und/oder Methanol in der Lage ist.
